# EUROPEAN PATENT SPECIFICATION

(11) **EP 3 892 723 B1**
(45) Date of publication and mention of the grant of the patent: **22.01.2025**
(21) Application number: 19893914.2
(22) Date of filing: 05.12.2019
(51) Int. Cl.: C12N 9/02, C12N 9/96

(54) **BILIRUBIN OXIDASE ACTIVITY IMPROVING METHOD, AND PRODUCT COMPRISING BILIRUBIN OXIDASE**
VERFAHREN ZUR VERBESSERUNG DER AKTIVITÄT VON BILIRUBIN-OXIDASE UND PRODUKT ENTHALTEND BILIRUBIN-OXIDASE
PROCÉDÉ D'AMÉLIORATION DE L'ACTIVITÉ DE LA BILIRUBINE OXYDASE ET PRODUIT COMPRENANT DE LA BILIRUBINE OXYDASE

(30) Priority: 05.12.2018 JP 2018228207
(43) Date of publication of application: 13.10.2021
(73) Proprietor: Ozeki Corporation, Nishinomiya-shi, Hyogo 663-8227 (JP)
(72) Inventor: KADO, Haruka, Nishinomiya-shi, Hyogo 663-8227 (JP); YAMADA, Hiroyuki, Nishinomiya-shi, Hyogo 663-8227 (JP); TSUBOI, Hirokazu, Nishinomiya-shi, Hyogo 663-8227 (JP); BOGAKI, Takayuki, Nishinomiya-shi, Hyogo 663-8227 (JP); KODA, Akio, Nishinomiya-shi, Hyogo 663-8227 (JP); TSUJINO, Yoshio, Kobe-shi, Hyogo 658-0003 (JP)
(74) Representative: Dr. Schön, Neymeyr & Partner Patentanwälte mbB
(86) International application number: PCT/JP2019/047551
(87) International publication number: WO 2020/116547

(56) References cited:
- WO-A1-2010/061822
- WO-A1-2018/141017
- JP-A- 2000 083 661
- JP-A- 2000 253 873
- JP-A- 2006 042 757
- JP-A- H06 284 886
- JP-A- H1 042 869
- JP-A- H1 042 869
- JP-A- S60 151 561
- US-A1- 2009 053 582
- TRIANTAFYLLOU A.Ö. ET AL.: "How do additives affect enzyme activity and stability in nonaqueous media?", BIOTECHNOL. BIOENGINEER., vol. 54, no. 1, 5 April 1997 (1997-04-05), pages 67 - 76, XP071151322

## Description

### Technical Field

The present invention relates to a method for improving bilirubin oxidase activity (hereinafter, referred to as "activity improving method"). The present invention relates to a bilirubin oxidase product for improving bilirubin oxidase activity. Further, the present invention relates to a method for producing a highly active bilirubin oxidase.

### Background Art

Bilirubin oxidase (herein, abbreviated as "BOD" as appropriate) is an enzyme that catalyzes a reaction which oxidizes bilirubin to biliverdin. BOD has been used for, for example, measuring the concentration of bilirubin in biological samples in the field of clinical laboratories. Since BOD has the property of oxidizing indole analogues, application of BOD to a hair dye has been expected.

Currently commercially supplied BOD products include, in addition to a BOD, (a) a buffering agent that makes pH suitable for an enzymatic reaction, and as needed, (b)(b-1) a component, such as a surfactant, that controls a reaction condition and (b-2) a common excipient such as a sugar and/or an inactive protein, and/or the like. The currently commercially supplied BOD products are distributed in a state in which the above-described component(s) is/are added to the BOD and freeze-dried together. The BOD in a dry state is dissolved in a predetermined solvent containing a surfactant and/or a buffering agent, when used. The BOD contained in such a product is relatively stable in a dry state. However, the BOD has a problem in that the BOD once dissolved tends to be deactivated in a solution state. For example, a commercially available BOD reagent is stable only for 96 hours when stored at 5°C in a tris-H₂SO₄ (pH 8.5) buffer.

Moreover, the BOD in the dry state requires an operation of dissolving a necessary amount of the BOD every time the BOD is used. Therefore, there are also problems such as an increased number of steps in using the BOD, and worker exposure of the BOD due to scattering of BOD powder in using the BOD. In addition, it is desirable that the BOD product be distributed at normal temperature from the viewpoint of distribution cost.

In view of the above circumstances, a method for stably storing a BOD in a BOD product (particularly in a solution state) has been studied. For example, Patent Literature 1 discloses a BOD stabilizing method with use of a reducing agent (sodium thiosulfate).

### Citation List

### [Patent Literature]

[Patent Literature 1]
Japanese Patent Application Publication Tokukaihei No. 10-42869 (Publication date: February 17, 1998)

### Summary of Invention

### Technical Problem

As described above, BOD is an enzyme that is strongly expected to be applied in industries. The inventors of the present application have set their own technical goal of simply improving BOD activity and decided to examine means to achieve the goal. In other words, an objective of an embodiment of the present invention is to provide means which allows for improvement of BOD activity. Specifically, an embodiment of the present invention provides a method for improving BOD activity, a BOD product allowing for improvement of BOD activity, and a method for producing a highly active bilirubin oxidase.

Note that Patent Literature 1 discloses a technique for solving a technical problem of stably storing BOD, and that this technique is not an invention for solving a technical problem of improving BOD activity.

### Solution to Problem

As a result of diligent studies for solving the above problem, the inventors of the present invention have found that the above-described problem can be solved by leaving, in a deoxygenated atmosphere, a BOD solution containing a BOD and a specific substance ("activity improving agent" described later). As a result, the inventors of the present invention have accomplished the present invention.

That is, an embodiment of the present invention is a method for improving bilirubin oxidase activity, the method including the step of leaving, in a deoxygenated atmosphere, a bilirubin oxidase solution containing a bilirubin oxidase and an activity improving agent, as defined in claim 1, the activity improving agent having an ABTS initial absorbance (A₄₃₆) reduction rate (%) of not less than 0.10% and a DMBQ initial absorbance (A₂₅₆) reduction rate (%) of not more than 10.00%.

Another embodiment of the present invention is a bilirubin oxidase product including: a container; and a bilirubin oxidase and an activity improving agent in the container, the container having an inner atmosphere which is deoxygenated, and the activity improving agent being defined as in claim 3 and having an ABTS initial absorbance (A₄₃₆) reduction rate (%) of not less than 0.10% and a DMBQ initial absorbance (A₂₅₆) reduction rate (%) of not more than 10.00%.

Herein described is a method for producing a highly active bilirubin oxidase, the method including the step of leaving, in a deoxygenated atmosphere, a bilirubin oxidase solution containing a bilirubin oxidase and an activity improving agent, the activity improving agent having an ABTS initial absorbance (A₄₃₆) reduction rate (%) of not less than 0.10% and a DMBQ initial absorbance (A₂₅₆) reduction rate (%) of not more than 10.00%.

### Advantageous Effects of Invention

An embodiment of the present invention advantageously makes it possible to improve BOD activity.

### Brief Description of Drawings

Fig. 1 is a graph showing changes in BOD activity when the following BOD solution (i) or (ii) was left in a specific temperature environment for a certain period of time: (i) a BOD solution to which no activity improving agent in accordance with an embodiment of the present invention had been added or (ii) a BOD solution containing the activity improving agent in accordance with an embodiment of the present invention.
Fig. 2 shows an ABTS initial absorbance (A₄₃₆) reduction rate (%) of an activity improving agent in accordance with an embodiment of the present invention or a comparative substance.
Fig. 3 shows a DMBQ initial absorbance (A₂₅₆) reduction rate (%) of an activity improving agent in accordance with an embodiment of the present invention or a comparative substance.

### Description of Embodiments

An embodiment of the present invention will be discussed below. Note, however, that the present invention is not limited to such an embodiment. The present invention is not limited to arrangements described below, but can be altered within the scope of the claims. An embodiment or example derived from a combination of technical means disclosed in different embodiments or examples, respectively, is also encompassed in the technical scope of the present invention. Further, it is possible to form a new technical feature by combining the technical means disclosed in respective embodiments. Any numerical range expressed as "A to B" in the present specification means "not less than A and not more than B (i.e., a range from A to B which includes both A and B)" unless otherwise stated.

In the present specification, X in accordance with an embodiment of the present invention may be simply referred to as "the present X" (X is any unique noun such as "activity improving method", "enzyme solution", "BOD", "activity improving agent", and "method for producing a highly active BOD").

### [1. Technical idea of embodiment of present invention]

The inventors of the present invention have diligently studied a method for improving an enzymatic activity of BOD. As a result, surprisingly, the inventors of the present invention have made a novel finding that the activity of a BOD (hereinafter, also referred to as "BOD activity") can be improved by: using a substance having an ABTS initial absorbance (A₄₃₆) reduction rate (%) and a DMBQ initial absorbance (A₂₅₆) reduction rate (%) within specific ranges, respectively; and placing the BOD in an deoxygenated atmosphere. As a result, the inventors have accomplished the present invention.

### [2. Activity improving method]

The present activity improving method is a method according to which a BOD solution containing a BOD and an activity improving agent are left in a deoxygenated atmosphere. The activity improving agent here is a substance which has an ABTS initial absorbance (A₄₃₆) reduction rate (%) of not less than 0.10% and a DMBQ initial absorbance (A₂₅₆) reduction rate (%) of not more than 10.00%.

The present activity improving method has an advantage that BOD activity can be simply improved by using the above activity improving agent.

The term "BOD solution" is intended to mean a solution containing at least a BOD and an activity improving agent, unless otherwise stated in the present specification. Note that a substance other than the BOD and the activity improving agent may be contained in the BOD solution.

In the present specification, the wording "improving BOD activity" means improving BOD activity after leaving, in a deoxygenated atmosphere, the BOD solution containing the BOD and the activity improving agent, as compared with BOD activity prior to leaving, in the deoxygenated atmosphere, the BOD solution containing the BOD and the activity improving agent.

Note that BOD activity is often expressed in terms of an activity relative to a mass (activity/mass) of the BOD. Therefore, in some cases, the activity may be also referred to as "specific activity". In other words, the activity improving method is also referred to as a specific activity improving method, and the activity improving agent is also referred to as a specific activity improving agent.

The specific activity can be calculated as follows. First, an absorbance (A₂₈₀) of the BOD solution containing only the BOD, at a wavelength of 280 nm, is divided by the molecular absorption coefficient of 115,000. Then, a value obtained by this division is multiplied by the molecular weight of 66,000 of the BOD. Further, a value thus obtained is defined as a BOD protein concentration (mg/mL) of the BOD solution. The specific activity (U/mg) is obtained by measuring an enzymatic activity (U/mL) of the BOD by a method described later, and dividing the enzymatic activity by the BOD protein concentration described above.

In the present activity improving method, greater improvement of the BOD activity is more preferable. In the present activity improving method, when the BOD activity prior to leaving the BOD is 100%, the BOD activity is improved by preferably not less than 105%, more preferably not less than 120%, still more preferably not less than 150%, and particularly preferably not less than 200%, as compared with the BOD activity prior to leaving the BOD.

In the present activity improving method, a period of time for leaving the BOD solution containing the BOD and the activity improving agent is not particularly limited. It is only necessary that the BOD solution be left at least until the BOD activity is improved. In the present activity improving method, from the viewpoint of production efficiency, it is more preferable that a shorter period of time be required for leaving the BOD solution for the purpose of improving the BOD activity. In the present activity improving method, the period of time for leaving the BOD solution is, for example, preferably not more than 50 days, preferably not more than 40 days, and preferably not more than 30 days.

In the present activity improving method, the BOD solution is left at a temperature, which is not particularly limited as long as the BOD activity improves at that temperature. In the present activity improving method, from the viewpoint of production efficiency, it is preferable that the BOD solution be left in an environment at a temperature of not lower than 10°C. In the present activity improving method, the temperature at which the BOD solution is left is for example, preferably not lower than 30°C, preferably not lower than 40°C, or preferably not lower than 50°C. The above arrangement advantageously makes it possible: to further improve the BOD activity; and to improve the BOD activity in a short period of time. In the present activity improving method, the upper limit of the temperature at which the BOD solution is left is, preferably, a temperature at which the BOD does not deactivate. For example, it is preferable to leave the BOD solution in an environment at a temperature of not higher than 60°C.

In the present specification, briefly, the BOD activity is evaluated as follows. After the BOD is added to the solution containing an ABTS substrate, a resulting solution is reacted at 37°C. The BOD activity is evaluated by measuring an increase in absorbance of the solution at a wavelength of 436 nm over time during the above reaction. Specific methods for measuring and evaluating the BOD activity will be described later in Test Examples.

### (2-1) BOD

Bilirubin oxidases (BODs) to which the present activity improving method can be applied include: (1) all well-known BODs (non-recombinant BODs) each obtained from any organism; (2) recombinant BODs each obtained by expressing a gene encoding a BOD derived from any organism, in the body of any organism or in vitro; (3) mutant BODs each obtained by mutation, such as substitution, insertion, or deletion, of an amino acid sequence of a BOD derived from any organism; and (4) BODs each modified with any carrier, for the purposes of stabilizing a BOD and improving an enzymatic activity of the BOD. Examples of the organism include, but are not limited to, *Myrothecium* sp. (e.g., *Myrothecium verrucaria*), *Penicillium janthinellum, Bacillus licheniformis, Corynebacterium glutamicum, Escherichia coli, Agaricus bisporus, Schizophyllum commune, Ganoderma tsunodae,* Asteraceae plants, Alfalfa, *Schizosaccharomyces pombe, Pichia pastoris,* and *Aspergillus* sp. The organism from which a gene encoding a BOD is derived may be identical to or different from the organism in which the gene is introduced and expressed in order to obtain the BOD.

The BOD may be (1) a BOD obtained from *Myrothecium* sp. (non-recombinant BOD), (2) a mutant BOD obtained by mutation, such as substitution, insertion, or deletion, of an amino acid sequence of a BOD derived from *Myrothecium* sp., (3) a recombinant BOD obtained by expressing, intracellularly in any organism or in vitro, a gene encoding a BOD derived from *Myrothecium* sp., (4) a recombinant BOD obtained by: introducing a gene encoding a BOD, which has been derived from *Myrothecium* sp., into any organism after optimization of codons in accordance with a gene sequence of the organism; and expressing the gene in the organism. For example, the recombinant BOD may be (1) a BOD obtained by: introducing a gene encoding a BOD, which has been derived from any organism, into *Aspergillus* sp.; and expressing the gene in *Aspergillus* sp., or (2) a BOD obtained by: introducing a gene encoding a BOD, which has been derived from any organism, into *Aspergillus* sp. after optimization of codons in accordance with a gene sequence of *Aspergillus* sp.; and expressing the gene in *Aspergillus* sp. More specifically, the recombinant BOD may be (1) preferably, a BOD obtained by introducing a gene encoding a BOD, which has been derived from *Myrothecium* sp., into *Aspergillus* sp. and expressing the gene in *Aspergillus* sp., and (2) more preferably, a BOD obtained by: introducing a gene encoding a BOD, which has been derived from *Myrothecium* sp., into *Aspergillus* sp. after optimization of codons in accordance with a gene sequence of *Aspergillus* sp.; and expressing the gene in *Aspergillus* sp.

The concentration of the BOD used in the present activity improving method is not particularly limited, and may be appropriately set in accordance with an application of the BOD.

### (2-2) Activity improving agent

The present activity improving agent is a substance which is capable of improving the BOD activity when left in a deoxygenated atmosphere together with the BOD. The present activity improving agent can be also referred to as an activity improving auxiliary agent. The present activity improving agent has an ABTS initial absorbance (A₄₃₆) reduction rate (% = reduction rate of absorbance at a wavelength of 436 nm in one minute) of not less than 0.10% and a DMBQ initial absorbance (A₂₅₆) reduction rate (% = reduction rate of absorbance at a wavelength of 256 nm in one minute) of not more than 10.00%.

Activity improving agents include at least one or more substances selected from the group consisting of: (a) sodium sulfite, uric acid, glyoxylic acid, N-acetyl-L-cysteine, L-cysteine hydrochloride, L-ascorbic acid, and Trolox; and (b) peptones (such as casein peptones, meat peptones, myocardial peptones, gelatin peptones, and soybean peptones), fish extract, malt extract, and glutathione (reduced form), corn steep liquor, and soybean flour.

The above inorganic substances can be those available from Wako Pure Chemical Industries, Ltd. On the other hand, examples of the above organic substances include, but are not limited to, at least one or more substances selected from the group consisting of a malt extract manufactured by Kyokuto Pharmaceutical Industrial Co., Ltd., a fish extract powder manufactured by Kyokuto Pharmaceutical Industrial Co., Ltd., a yeast extract powder manufactured by Kyokuto Pharmaceutical Industrial Co., Ltd., Kyokuto Peptone manufactured by Kyokuto Pharmaceutical Industrial Co., Ltd., Peptone A manufactured by Kyokuto Pharmaceutical Industrial Co., Ltd., Particase manufactured by Kyokuto Pharmaceutical Industrial Co., Ltd., Potato Peptone CP manufactured by Kyokuto Pharmaceutical Industrial Co., Ltd., yeast extracts manufactured by Asahi Group Foods, Ltd. (e.g., Meast P1G and Yeastock S-Pd), corn steep liquor (also abbreviated as "CSL") manufactured by Oriental Yeast Co., Ltd., Hipolypepton manufactured by Nihon Pharmaceutical Co., Ltd., Soybean Flour 88 manufactured by Showa Sangyo Co., Ltd., Tryptone manufactured by Japan Becton, Dickinson and Company, Yeast Extract manufactured by Japan Becton, Dickinson and Company, and the like. The present activity improving method can further improve the BOD activity in a case where the present activity improving agent is at least one or more selected from the above-described group. Various types of the activity improving agent listed in the present specification may be each used alone, or a plurality of types may be used in combination.

In the present activity improving method, the optimum concentration of the activity improving agent contained in the BOD solution may vary depending on the concentration of the BOD in the BOD solution, the type of the activity improving agent, and the like. Therefore, the final concentration of the activity improving agent contained in the BOD solution may be appropriately set within a range which makes it possible to obtain an effect associated with an embodiment of the present invention.

### (2-3) Deoxygenated atmosphere

In the present activity improving method, in order to prevent oxidation of the BOD, it is preferable that the BOD be present in an atmosphere in which oxygen is present in an amount as small as possible (i.e., a deoxygenated atmosphere). On this account, in the present activity improving method, the BOD solution containing the BOD and the activity improving agent is left in the "deoxygenated atmosphere". In the present activity improving method, the term "deoxygenated atmosphere" is intended to mean a state in which an oxygen concentration of an atmosphere in which the BOD solution is left is lower than an oxygen concentration in a natural state (in other words, state at normal temperature and normal pressure).

The present activity improving method has an advantage that the BOD activity can be easily improved by leaving the BOD solution in the deoxygenated atmosphere.

In the present activity improving method, from the viewpoint of easily improving the BOD activity, the oxygen concentration of the atmosphere in which the BOD solution is left is preferably less than 0.5% by volume, and particularly preferably less than 0.1% by volume.

In the present activity improving method, there is no particular limitation on a method for achieving the deoxygenated atmosphere. For example, in the present activity improving method, the deoxygenated atmosphere can be achieved by: (1) placing a deoxygenating agent in a container in which the BOD solution is contained; (2) storing the BOD solution in a container made of a material having a component of a deoxygenating agent; (3) replacing oxygen in a container by an inert gas (nitrogen gas or a rare gas such as argon gas); or (4) leaving the BOD solution under vacuum or under a reduced pressure condition. These methods for achieving the deoxygenated atmosphere may be each used alone, or a plurality of those methods may be used in combination.

From the viewpoint of simplicity, the above method (1) is preferable. Examples of the deoxygenating agent used in the above method (1) include, but are not limited to, AGELESS (registered trademark) manufactured by Mitsubishi Gas Chemical Company, Inc., AGELESS OMAC (registered trademark) manufactured by Mitsubishi Gas Chemical Company, Inc., Anaero Pack (registered trademark) manufactured by Mitsubishi Gas Chemical Company, Inc., Everfresh manufactured by TORISHIGE SANGYO Co., Ltd., and WonderKeep (registered trademark) manufactured by Powdertech Co., Ltd.

Note that in the present activity improving method, from the viewpoint of more easily improving the BOD activity, it is preferable to have a low concentration (preferably less than 0.2 mg/L, and particularly preferably less than 0.04 mg/L) of dissolved oxygen in the BOD solution containing the BOD in addition to leaving the BOD solution in the deoxygenated atmosphere. The concentration of dissolved oxygen in the BOD solution is preferably not more than a saturated oxygen concentration of the solution in a case where the oxygen concentration of the atmosphere in which the BOD solution is stored is the above-described preferred oxygen concentration. Note that the saturated oxygen concentration of the solution may vary depending on the temperature of the solution. For example, when the oxygen concentration of an air atmosphere is 0.5% by volume, the saturated oxygen concentration of the solution is 0.2 mg/L at 25°C, and 0.35 mg/L at 0°C. It is possible to achieve a preferred aspect of the concentration of dissolved oxygen in the BOD solution (low dissolved oxygen state), by carrying out a well-known degassing operation on BOD solution.

### (2-4) ABTS initial absorbance reduction rate

In the present specification, the absorbance of a solution containing ABTS (ABTS solution), at a wavelength of 436 nm, is defined as an ABTS initial absorbance (A₄₃₆). In the present specification, the ABTS initial absorbance (A₄₃₆) reduction rate (%) of the activity improving agent is defined to be a reduction rate (%) of the absorbance of a mixed solution, which contains the above ABTS solution and the above activity improving agent, at a wavelength of 436 nm in one minute, with respect to the ABTS initial absorbance (A₄₃₆) (100%). Note that the ABTS solution is a solution in which the concentration of ABTS is adjusted such that the absorbance (A₄₃₆) at a wavelength of 436 nm is approximately 1.3 at an optical path length of 1.0 cm. The value "approximately 1.3" here is intended to mean a value in a range in which 1.3 is obtained by rounding the value to the first decimal place. Test Examples described later will explain specific methods for measuring the absorbance (A₄₃₆) of the ABTS solution at a wavelength of 436 nm and the ABTS initial absorbance (A₄₃₆) reduction rate (%) of the activity improving agent. ABTS is an abbreviation for 2,2'-azinobis(3-ethylbenzothiazoline-6-sulfonic acid).

In the present activity improving method, when the ABTS initial absorbance (A₄₃₆) reduction rate (%) of the activity improving agent is measured, the concentration of the activity improving agent in the ABTS solution for measurement is set as follows: (a) the concentration is set to 1 mM, when the molecular weight of the activity improving agent is evident as in a case where the activity improving agent is made of a single compound; and (b) when the activity improving agent is a substance which is made of a composition having an inevident molecular weight, the content of nitrogen element is used as a reference and the concentration is set such that the content of nitrogen element derived from the activity improving agent is 0.011 g in 100 mL of the ABTS solution for measurement. Note that, in the above (b), the content of nitrogen element derived from the activity improving agent in the ABTS solution is a value calculated by the Kjeldahl method.

In the present activity improving agent, it is only necessary that the ABTS initial absorbance (A₄₃₆) reduction rate (%) be not less than 0.10%. The ABTS initial absorbance (A₄₃₆) reduction rate (%) is preferably not less than 0.25%, more preferably not less than 0.50%, still more preferably not less than 1.00%, and particularly preferably not less than 3.00%. The above arrangement advantageously makes it possible to more easily improve the BOD activity.

### (2-5) DMBQ initial absorbance (A₂₅₆) reduction rate

In the present specification, the DMBQ initial absorbance (A₂₅₆) is defined to be the absorbance of a mixed solution at a wavelength of 256 nm immediately after adding the activity improving agent to a solution containing DMBQ (DMBQ solution). In the present specification, the DMBQ initial absorbance (A₂₅₆) reduction rate (%) of the activity improving agent is defined to be a reduction rate (%) of the absorbance of the mixed solution, which contains the above DMBQ solution and the above activity improving agent, at a wavelength of 256 nm in one minute, with respect to the DMBQ initial absorbance (A₂₅₆) (100%). The DMBQ solution is a solution in which the density of DMBQ is adjusted such that the absorbance (A₂₅₆) at a wavelength of 256 nm is approximately 1.8 at an optical path length of 1.0 cm. The value "approximately 1.8" here is intended to mean a value in a range in which 1.8 is obtained by rounding the value to the first decimal place. Test Examples described later will explain specific methods for measuring the absorbance (A₂₅₆) of the DMBQ solution at a wavelength of 256 nm and the DMBQ initial absorbance (A₂₅₆) reduction rate (%) of the activity improving agent. DMBQ is an abbreviation for 2,5-dimethyl-1,4-benzoquinone.

In the present activity improving method, when the DMBQ initial absorbance (A₂₅₆) reduction rate (%) of the activity improving agent is measured, the concentration of the activity improving agent in the DMBQ solution for measurement is set as follows: (a) the concentration is set to 0.1 mM, when the molecular weight of the activity improving agent is evident as in a case where the activity improving agent is made of a single compound; and (b) when the activity improving agent is a substance which is made of a composition having an inevident molecular weight, the content of nitrogen element is used as a reference and the concentration is set such that the content of nitrogen element derived from the activity improving agent is 0.0011 g in 100 mL of the DMBQ solution for measurement. Note that, in the above (b), the content of nitrogen element derived from the activity improving agent in the DMBQ solution is a value calculated by the same method as that in the above section (2-4).

In the present activity improving agent, it is only necessary that the DMBQ initial absorbance (A₂₅₆) reduction rate (%) be 10.00% or less. The above DMBQ initial absorbance (A₂₅₆) reduction rate (%) is preferably not more than 7.00%, more preferably not more than 5.00%, still more preferably not more than 4.00%, still more preferably not more than 1.00%, and particularly preferably not more than 0.00%, that is, a detection limit or lower in the method for measuring in the present specification. The above arrangement advantageously makes it possible to more easily improve the BOD activity.

### (2-6) Solvent

In the present activity improving method, the BOD is contained in a solution together with the activity improving agent. There is no particular limitation on a solvent for forming the BOD solution as long as the solvent can dissolve the BOD and the present activity improving agent, and any conventional well-known solvent can be used as the solvent. The present solvent is preferably water (e.g., distilled water) from the viewpoint of easily dissolving the present BOD and the present activity improving agent.

The present BOD solution may have any pH depending on an application for which the present BOD solution is used. It is preferable that the present BOD solution have a pH of pH 6 to pH 10 so that a steric structure of the BOD contained in the BOD solution will be kept stable. It is preferable that the present BOD solution include a buffering agent so as to have a desired pH. It is possible to use, as the above buffering agent, a conventional well-known buffering agent. Further, in order to allow the present BOD solution to have a desired pH, the pH of the present BOD solution can be adjusted by a conventional well-known method using a conventional well-known acid or alkali.

### (2-7) Others

The present BOD solution may contain any substance in addition to the BOD, the activity improving agent, and the solvent, as long as the BOD activity can be improved. The present BOD solution may include, for example, buffering agents, acids, bases, inorganic salts, organic salts, metal salts, sugars, amino acids, inactive proteins, peptides, fatty acids, lipids, surfactants, and resins.

### (2-8) Applications

The present BOD solution, which has been left according to the present activity improving method and has an improved BOD activity, can be used in desired applications. The present BOD solution, which has the BOD activity having been improved by the present activity improving method, can be used in, for example, hair dyeing, measuring the concentration of bilirubin in biological samples, removing bilirubin in biological samples, cosmetics, food processing, enzyme fuel cells (e.g., enzymatic biofuel cells, and enzymatic batteries), and crosslinking proteins.

### [3. BOD products]

Another embodiment of the present invention provides a BOD product which contains a BOD and an activity improving agent in a container having an inner atmosphere which is deoxygenated. The activity improving agent has an ABTS initial absorbance (A₄₃₆) reduction rate (%) of not less than 0.10% and a DMBQ initial absorbance (A₂₅₆) reduction rate (%) of not more than 10.00%.

Since the present BOD product has the above arrangement, the present BOD product advantageously makes it possible to improve the BOD activity which is contained in the BOD product.

Although the BOD and the activity improving agent, which are contained in the present BOD product, may be in the form of solid such as powder, it is preferable that the BOD and the activity improving agent be in the form of solution (that is, the BOD solution) obtained by dissolving the BOD and the activity improving agent in an appropriate solvent. Although BOD is unstable in a solution state, the present BOD product can improve the BOD activity even in a case where the BOD is in a solution state.

In a case where the BOD and the activity improving agent, which are contained in the present BOD product, are in a solution state, there are the following advantages: (a) no manipulation is required to dissolve the BOD and the activity improving agent every time they are used; and (b) there is no risk of worker exposure of the BOD due to scattering of powder of the BOD when the BOD is used.

Note that the section [2. Activity improving method] can be referred to for explanations of the BOD, the activity improving agent, the solvent and the like which are contained in the present BOD product. Moreover, the present BOD product may further include any of the substances described in the above section (2-7) Others. In addition, in the present BOD product, the deoxygenated atmosphere in the container can be achieved by the method for forming a deoxygenated atmosphere described in the above section (2-3) Deoxygenated atmosphere.

### (3-1) Container

The container, which can be used in the present BOD product, is not limited in material, shape, size, etc. as long as the container is at least capable of storing therein the BOD and the activity improving agent. The container may be a container capable of keeping a deoxygenated state in the container for a certain period of time, or a container capable of producing the deoxygenated atmosphere inside the container. The container may be made of glass or resin. In addition, the container may have a bottle shape or a bag shape. It is possible to use, as the container having a bag shape, a resin bag which has a fastener (for example, Ziploc (Registered Trademark)). The container may have a size which is appropriately designed depending on the volume of the BOD and the activity improving agent which are to be stored or the volume of the BOD solution which is to be stored.

In order to easily keep the deoxygenated state for a long period of time, the present container is preferably made of a material which is impermeable to oxygen (or a material which is hardly permeable to oxygen). Moreover, the present container may be made of a material which contains a component having a property of the deoxygenating agent. In addition, in order to make it possible to further improve the BOD activity, the present container is preferably a semi-sealed container, and more preferably a sealed container which is impermeable to any substances.

In the present BOD product, the BOD solution may be obtained by (i) adding the solvent in the container which contains the BOD and the activity improving agent and (ii) dissolving the BOD and the activity improving agent. Therefore, it is preferable that the container in the present BOD product be resistant to the above solvent.

### (3-2) Example of specific aspect of present BOD product

Although there is no particular limitation on an aspect of the present BOD product, an aspect (Aspect A) of the present BOD product may include, for example: a container that has an openable and closable cover and that includes a packaged deoxygenating agent on an inner side of the cover; and the BOD and the activity improving agent which are stored in the container. In the case of Aspect A, the BOD and the activity improving agent, which are stored in the container, may be in the form of liquid or solid. Assume a case where the BOD and the activity improving agent in the form of solid are stored in the container in Aspect A. In this case, even after the BOD solution is obtained by adding the solvent to the container and dissolving the BOD and the activity improving agent, the BOD activity can be improved by keeping the BOD solution left in the deoxygenated atmosphere in the above container in which the BOD and the activity improving agent has been stored.

In a case where the BOD and the activity improving agent are in the form of solid such as powder, it is not necessary to provide the packaged deoxygenating agent on the cover. In this case, the deoxygenating agent can be contained together with the BOD and the activity improving agent in the container. The deoxygenating agent in the container causes the atmosphere inside the container to be the deoxygenated atmosphere. Further, the air in the container may be replaced with an inert gas such as nitrogen gas.

Alternatively, in another aspect of the present BOD product, the BOD, the activity improving agent, and the packaged deoxygenating agent are stored in the resin bag which has a fastener.

### (3-3) Applications

The present BOD product can be used in desired applications. The present BOD product can be used in, for example, hair dyeing, measuring the concentration of bilirubin in biological samples, removing bilirubin in biological samples, cosmetics, food processing, enzyme fuel cells (e.g., enzymatic biofuel cells, and enzymatic batteries), and crosslinking proteins.

### [4. Method for producing highly active BOD] (not forming part of the invention)

Herein described is a -A-method for producing a highly active BOD which includes the step of leaving, in a deoxygenated atmosphere, a BOD solution containing a BOD and an activity improving agent. The activity improving agent here is a substance which has an ABTS initial absorbance (A₄₃₆) reduction rate (%) of not less than 0.10% and a DMBQ initial absorbance (A₂₅₆) reduction rate (%) of not more than 10.00%.

With the above arrangement, the method for producing a highly active BOD advantageously makes it possible to provide a highly active BOD having an improved BOD activity. The highly active BOD is a BOD having a specific activity which has been increased by carrying out the above method for producing a highly active BOD, as compared with the BOD prior to carrying out this method.

The method for producing a highly active BOD is also referred to simply as the present production method.

The section [2. Activity improving method] can be referred to for explanations of the BOD, the activity improving agent, the solvent and the like in the present production method. In addition, in the present production method, it is possible to further use any of the substances described in the above section (2-7) Others. In addition, in the present production method, the deoxygenated atmosphere can be formed by the method for forming a deoxygenated atmosphere described in the above section (2-3) Deoxygenated atmosphere.

The present production method may further include the step of obtaining the BOD in the form of solid (powder) by freeze-drying the BOD solution after the step of leaving the BOD solution. When the present production method includes the step of obtaining the BOD in the form of solid, the present production method can provide the highly active BOD in the form of solid.

The highly active BOD obtained by the present production method can be used in various applications as described in the above section (2-8) Applications.

### Examples

The following will specifically discuss an embodiment of the present invention by using Examples. Note, however, that the present invention is not limited to such Examples (Test Examples).

The present activity improving agent and other substances (hereinafter referred to as "comparative substance(s)"), which were used in the present Examples, are as follows. Note that only glyoxylic acid was adjusted to pH 7.0 by using a potassium hydroxide solution, and used in the present Examples.

### (Activity improving agent)

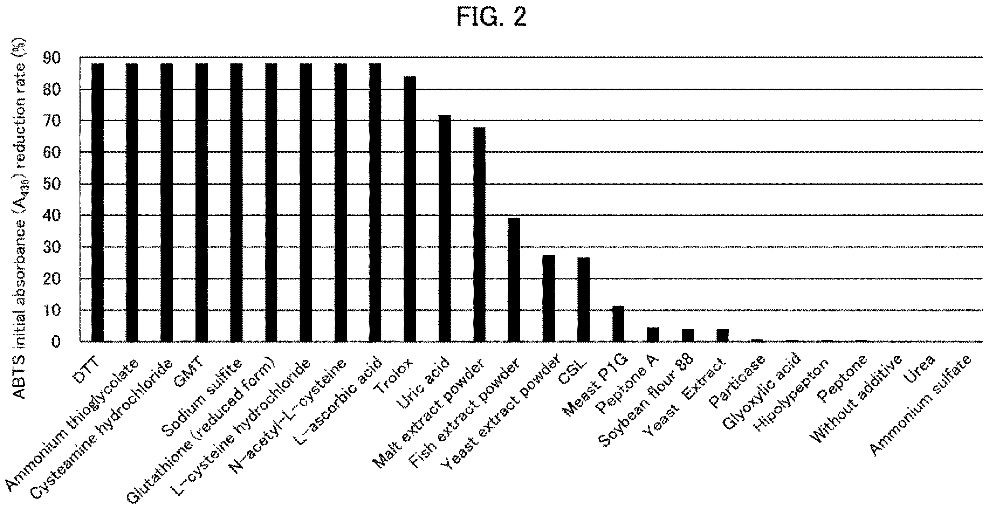

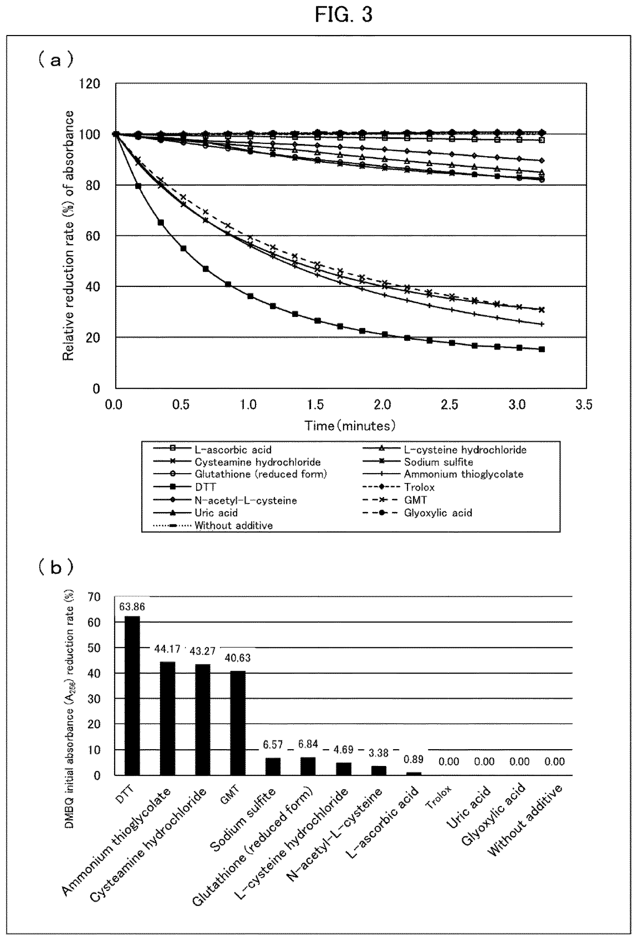

- uric acid (manufactured by Wako Pure Chemical Industries, Ltd.)
- glyoxylic acid (manufactured by Wako Pure Chemical Industries, Ltd.)
- malt extract powder (manufactured by Kyokuto Pharmaceutical Industrial Co., Ltd.)
- fish extract powder (manufactured by Kyokuto Pharmaceutical Industrial Co., Ltd.)
- yeast extract powder (manufactured by Kyokuto Pharmaceutical Industrial Co., Ltd.)
- CSL, Solulys 095E (manufactured by Oriental Yeast Co., Ltd.) (expressed as CSL in Tables 1, 3, 7, and 8, and Figs. 2 and 3)
- Meast P1G (Asahi Group Foods, Ltd.)
- Peptone A (manufactured by Kyokuto Pharmaceutical Industrial Co., Ltd.)
- Soybean Flour 88 (manufactured by Showa Sangyo Co., Ltd.)
- Yeast Extract (manufactured by Japan Becton, Dickinson and Company)
- Particase (manufactured by Kyokuto Pharmaceutical Industrial Co., Ltd.)
- Hipolypepton (manufactured by Nihon Pharmaceutical Co., Ltd.)
- peptone (manufactured by Kyokuto Pharmaceutical Industrial Co., Ltd.)
- sodium sulfite (manufactured by Wako Pure Chemical Industries, Ltd.)
- glutathione (reduced form) (manufactured by Wako Pure Chemical Industries, Ltd.)
- L-cysteine hydrochloride (manufactured by Wako Pure Chemical Industries, Ltd.)
- N-acetyl-L-cysteine (manufactured by Wako Pure Chemical Industries, Ltd.)
- L-ascorbic acid (manufactured by Wako Pure Chemical Industries, Ltd.)
- Trolox (manufactured by Sigma-Aldrich Co. LLC)

### (Comparative substance)

- urea (manufactured by Wako Pure Chemical Industries, Ltd.)
- ammonium sulfate (manufactured by Nacalai Tesque, Inc.)
- dithiothreitol (DTT) (manufactured by Wako Pure Chemical Industries, Ltd.)
- ammonium thioglycolate (manufactured by Sasaki Chemical Co., Ltd.)
- cysteamine hydrochloride (manufactured by Sasaki Chemical Co., Ltd.)
- glyceryl monothioglycolate (abbreviated as GMT in Tables 1, 2, 7, and 8, and Figs. 2 and 3) (manufactured by Sasaki Chemical Co., Ltd.)

### [Test Example 1] Improvement of BOD activity by present activity improving method

A BOD solution, which contains: a BOD; and the present activity improving agent or a comparative substance, was left in an environment at a temperature of 30°C in a deoxygenated atmosphere for a certain period of time. After the BOD solution was left as above, improvement of the BOD activity by the present activity improving method was evaluated by measurement of the BOD activity.

In Test Example 1, a BOD gene derived from a *Myrothecium verrucaria* MT-1 (GenBank accession number: D12579.1) was introduced into an *Aspergillus* sp., and then a recombinant BOD (hereinafter, also referred to simply as "BOD") expressed in the *Aspergillus* sp. was purified by using a well-known technique and then used as the BOD. The BOD gene derived from the *Myrothecium verrucaria* MT-1 was codon-optimized in accordance with a gene sequence of the *Aspergillus* sp., and then introduced into the *Aspergillus* sp. Further, the BOD thus purified was dissolved in a 50 mM glycine-KOH (pH 8.5) buffer so that the concentration of the BOD became 1.0 mg/mL. Then, a resultant solution was used as a BOD enzyme solution.

The enzymatic activity of the BOD was measured as follows. To 1780 pL of a 50 mM potassium phosphate buffer (pH 6.5), 200 pL of a 20 mM ABTS substrate solution was added, so that a mixed solution (1980 pL) was prepared. This mixed solution was incubated for 1 min at 37°C. Thereafter, 20 pL of a solution containing the BOD (specifically, a BOD solution for evaluation, which will be described later) was added to the mixed solution, and a resultant mixed solution (2 mL) was incubated at 37°C. While the mixed solution was incubated, the absorbance of the mixed solution at a wavelength of 436 nm was measured immediately after that addition of the BOD solution for evaluation (at 0 seconds) and thereafter every 20 seconds. On the basis of (i) the absorbance of the mixed solution at a wavelength of 436 nm immediately after the addition of the BOD solution for evaluation and (ii) the absorbance of the mixed solution at a wavelength of 436 nm after a lapse of 1 minute from the addition of the BOD solution for evaluation, calculation was carried out for obtaining an increase rate per minute of the absorbance of the mixed solution at a wavelength of 436 nm after the addition of the BOD solution for evaluation. The BOD activity (unit) in the BOD solution for evaluation was calculated from the increase rate. In the present specification, the amount of enzyme for oxidizing 1 pmol of ABTS per minute was defined as one unit.

A specific test method of Test Example 1 is as follows. To a BOD enzyme solution containing a BOD, one of the above-described present activity improving agents was added, so that a BOD solution (which is the BOD solution according to an embodiment of the present invention) was prepared. Further, one of the above-described comparative substances was added to the BOD enzyme solution, so that another BOD solution was prepared. In addition, still another BOD solution was prepared by adding, to the BOD enzyme solution, distilled water in place of the activity improving agent or the comparative substance, so that a control (denoted as Without additive in Tables 1 to 3) was prepared. The final concentration of the activity improving agent or the comparative substance in each of the above BOD solutions was shown in "mM" or "percentage (%)" (amount (g) of the activity improving agent or the comparative substance relative to 100 mL of the BOD solution) in Table 1.

**[Table 1]**

| Final concentration of activity improving agent or comparative substance used in each BOD solution | |
|---|---|
| Activity improving agent or comparative substance | Final concentration |
| Without additive | - |
| Urea | 40 mM |
| Ammonium sulfate | 40 mM |
| DTT | 20 mM |
| Ammonium thioglycolate | 20 mM |
| Cysteamine hydrochloride | 20 mM |
| GMT | 20 mM |
| Sodium sulfite | 40 mM |
| Glutathione (reduced form) | 20 mM |
| L-cysteine hydrochloride | 8 mM |
| N-acetyl-L-cysteine | 8 mM |
| L-ascorbic acid | 8 mM |
| Trolox | 40 mM |
| Uric acid | 40 mM |
| Malt extract powder | 8.80(%) |
| Fish extract powder | 7.34(%) |
| Yeast extract powder | 4.00(%) |
| CSL | 6.30(%) |
| Meast P1G | 2.00(%) |
| Peptone A | 4.40(%) |
| Soybean Flour 88 | 4.00(%) |
| Yeast Extract | 2.00(%) |
| Particase | 4.40(%) |
| Glyoxylic acid | 40 mM |
| Hipolypepton | 4.00(%) |
| Peptone | 3.66(%) |

Thereafter, one of these BOD solutions and a deoxygenating agent (Anaero Pack manufactured by Mitsubishi Gas Chemical Company, Inc.) were included in one container. Then, the container was sealed, so that a BOD container was prepared. The BOD container was left in an environment at a temperature of 30°C in a deoxygenated atmosphere for 47 days (Table 2) or 14 days (Table 3). After leaving the BOD container under such a condition, the BOD solution was collected from the BOD container and used as a BOD solution for evaluation.

The BOD activity (unit) in the BOD solution for evaluation was measured by the above-described method. With regard to the BOD activity, calculation was carried out for obtaining an activity (%), which was defined to be a relative value of the BOD activity (unit) after leaving the BOD solution for a predetermined number of days, on the assumption that the BOD activity (unit) at the start of leaving the BOD solution (day 0) was 100%. Table 2 shows results of a case where the BOD solution was left for 47 days, and Table 3 shows results of a case where the BOD solution was left for 14 days.

**[Table 2]**

| Activity improving agent or comparative substance | Activity (%) | |
|---|---|---|
| | Day 0 | Day 47 |
| Without additive | 100 | 83 |
| DTT | 100 | 0 |
| Ammonium thioglycolate | 100 | 0 |
| Cysteamine hydrochloride | 100 | 1 |
| GMT | 100 | 1 |
| Sodium sulfite | 100 | 162 |
| Glutathione (reduced form) | 100 | 113 |
| L-cysteine hydrochloride | 100 | 130 |
| N - acetyl- L- cysteine | 100 | 122 |
| L-ascorbic acid | 100 | 151 |
| Trolox | 100 | 251 |
| Uric acid | 100 | 155 |

**[Table 3]**

| Activity improving agent or comparative substance | Activity (%) | |
|---|---|---|
| | Day 0 | Day 14 |
| Without additive | 100 | 59 |
| Urea | 100 | 56 |
| Ammonium sulfate | 100 | 65 |
| Uric acid | 100 | 156 |
| Malt extract powder | 100 | 149 |
| Fish extract powder | 100 | 132 |
| Yeast extract powder | 100 | 111 |
| CSL | 100 | 168 |
| Meast P1G | 100 | 121 |
| Peptone A | 100 | 119 |
| Soybean Flour 88 | 100 | 146 |
| Yeast Extract | 100 | 105 |
| Particase | 100 | 136 |
| Glyoxylic acid | 100 | 194 |
| Hipolypepton | 100 | 122 |
| Peptone | 100 | 119 |

It has been found from Tables 2 and 3 that in a case where the BOD solution containing the present activity improving agent is left in the deoxygenated atmosphere, the BOD in this BOD solution which has been left has a higher enzymatic activity as compared with the BOD in this BOD solution which has not been left yet. In other words, it has been found that the present activity improving method can improve the BOD activity. On the other hand, in the case of the control, it has been found that the BOD in the BOD solution which has been left has a lower enzymatic activity as compared with the BOD in this BOD solution which has not been left yet. Further, it has been also found that in a case where the BOD solution containing the comparative substance is left, the BOD in this BOD solution which has been left exhibits (1) a lower enzymatic activity as compared with this BOD solution which has not been left yet and (2) substantially the same or lower enzymatic activity as compared with the control even after the BOD solution has been left in the deoxygenated atmosphere. That is, it has been found that the comparative substance cannot improve the BOD activity.

In Test Example 1, the BOD container was obtained by: including, in one container, (a) the BOD solution containing (a-1) the BOD enzyme solution containing the BOD and (a-2) one of the present activity improving agents, and (b) the deoxygenating agent; and sealing this container. This BOD container can be also called a BOD product in accordance with an embodiment of the present invention. Further, in a case where a BOD was obtained after leaving the BOD solution containing the present activity improving agent in the deoxygenated atmosphere, the BOD thus obtained had an increased specific activity as compared with the BOD which had not been left yet. Therefore, the BOD thus obtained can be also called a highly active BOD. In other words, Test Example 1 can be also said to be implementation of the present activity improving method. Further, Test Example 1 can be also said to be production of the present BOD product. In addition, Test Example 1 can be also said to be implementation of a method for producing a highly active bilirubin oxidase in accordance with an embodiment of the present invention.

### (Test Example 2) Studies on temperature environment in leaving BOD solution in present activity improving method

Studies were made on a temperature environment in leaving, in the deoxygenated atmosphere, the BOD solution containing the BOD and the present activity improving agent.

The following describes a specific test method in Test Example 2. A BOD solution containing an activity improving agent was prepared as in Test Example 1. Further, another BOD solution which contained no activity improving agent (to which no activity improving agent was added) was prepared and used as a control. In Test Example 2, L-ascorbic acid or uric acid was used as the activity improving agent. Moreover, the final concentration of L-ascorbic acid in the BOD solution was set to 4 mM, and the final concentration of uric acid in the BOD solution was set to 20 mM. In addition, in Test Example 2, the concentration of the BOD in the BOD enzyme solution was set to 3.0 mg/mL.

Each of the above BOD solutions and a deoxygenating agent (Anaero Pack·Kenki manufactured by Mitsubishi Gas Chemical Company, Inc.) were included in one container (pouch) which was not permeable to oxygen. Then, the container was sealed, so that a BOD container was prepared. The BOD container was left at room temperature (approximately 25°C) for approximately 2 hours, so that an inner atmosphere of the container became the deoxygenated atmosphere. Thereafter, the BOD container was left in a thermostatic chamber set at 5°C, 10°C, 20°C, 30°C, 40°C, or 50°C for a certain period of time. Here, (a) the BOD solution without any additive was left for 1 day, 3 days, 7 days, 14 days, or 29 days, (b) the BOD solution containing L-ascorbic acid was left for 1 day, 3 days, 7 days, or 14 days, and (c) the BOD solution containing uric acid was left for 1 day, 3 days, 8 days, 14 days, or 28 days. Three samples (BOD containers) were prepared for each condition. After leaving the BOD containers under the above conditions, the BOD solution was collected from each of the BOD containers and used as a BOD solution for evaluation.

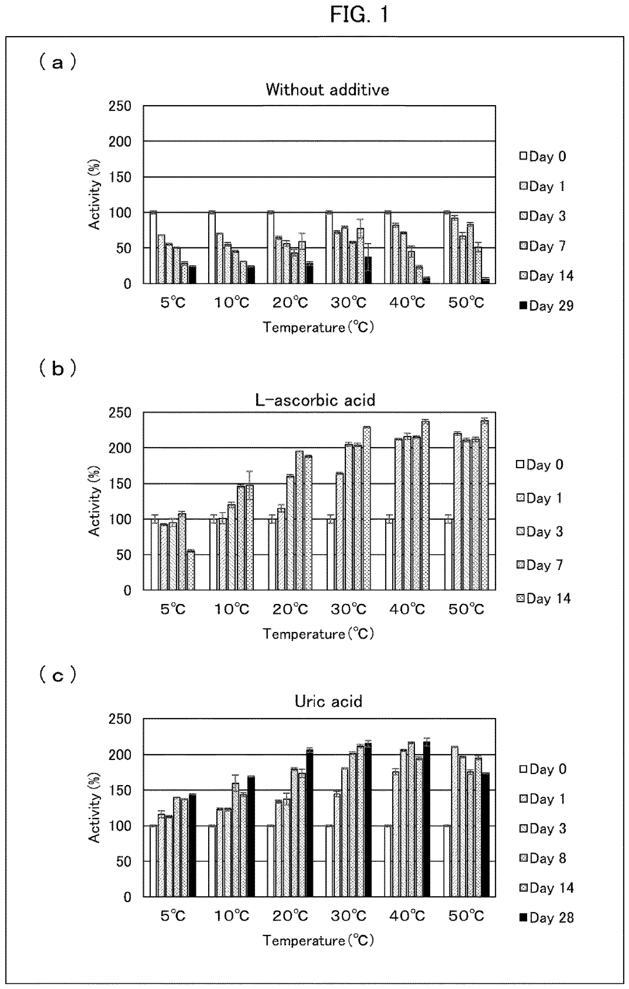

The BOD activity (unit) in the BOD solution for evaluation was measured by the above-described method. With regard to the BOD activity, calculation was carried out for obtaining an activity (%), which was defined to be a relative value of the BOD activity (unit) after leaving the BOD solution for a predetermined number of days, on the assumption that the BOD activity (unit) at the start of leaving the BOD solution (day 0) was 100%. Tables 4 to 6 show a mean value of each set of the three samples. Table 4 shows results in the case of the control. Table 5 shows results in a case where L-ascorbic acid was used as the activity improving agent. Table 6 shows results in a case where uric acid was used as activity improving agent. Further, Fig. 1 shows a mean value and a standard error for each set of the three samples.

**[Table 4]**

| Without additive | | | | | | |
|---|---|---|---|---|---|---|
| | Activity (%) | | | | | |
| Temperature | Day 0 | Day 1 | Day 3 | Day 7 | Day 14 | Day 29 |
| 5°C | 100 | 68 | 55 | 50 | 28 | 24 |
| 10°C | 100 | 70 | 55 | 45 | 31 | 24 |
| 20°C | 100 | 64 | 56 | 43 | 59 | 28 |
| 30°C | 100 | 72 | 79 | 58 | 77 | 37 |
| 40°C | 100 | 82 | 71 | 45 | 23 | 7 |
| 50°C | 100 | 92 | 67 | 83 | 51 | 6 |

**[Table 5]**

| L-ascorbic acid | | | | | |
|---|---|---|---|---|---|
| | Activity (%) | | | | |
| Temperature | Day 0 | Day 1 | Day 3 | Day 7 | Day 14 |
| 5°C | 100 | 92 | 95 | 107 | 55 |
| 10°C | 100 | 101 | 120 | 146 | 147 |
| 20°C | 100 | 115 | 160 | 195 | 188 |
| 30°C | 100 | 164 | 205 | 204 | 229 |
| 40°C | 100 | 212 | 216 | 215 | 237 |
| 50°C | 100 | 220 | 211 | 212 | 238 |

**[Table 6]**

| Uric acid | | | | | | |
|---|---|---|---|---|---|---|
| | Activity (%) | | | | | |
| Temperature | Day 0 | Day 1 | Day 3 | Day 8 | Day 14 | Day 28 |
| 5°C | 100 | 116 | 113 | 140 | 137 | 144 |
| 10°C | 100 | 123 | 123 | 159 | 144 | 169 |
| 20°C | 100 | 134 | 137 | 179 | 173 | 206 |
| 30°C | 100 | 144 | 181 | 202 | 212 | 215 |
| 40°C | 100 | 176 | 206 | 216 | 194 | 217 |
| 50°C | 100 | 210 | 196 | 175 | 195 | 174 |

Fig. 1 is a graph showing changes in the BOD activity when the following BOD solution (i) or (ii) was left in a specific temperature environment for a certain period of time: (i) the BOD solution to which no activity improving agent in accordance with an embodiment of the present invention had been added or (ii) the BOD solution containing the activity improving agent in accordance with an embodiment of the present invention. (a) of Fig. 1 shows results for the BOD solution to which no present activity improving agent had been added, (a) of Fig. 1 shows, in a graph, the contents of Table 4. (b) of Fig. 1 shows results for the BOD solution to which L-ascorbic acid had been added as the present activity improving agent, (b) of Fig. 1 shows, in a graph, the contents of Table 5. (c) of Fig. 1 shows results for the BOD solution to which uric acid had been added as the present activity improving agent, (c) of Fig. 1 shows the contents of Table 6 in a graph. With regard to the control, no improvement in the BOD activity was found from Tables 4 to 6 and Fig. 1, since the BOD activity decreased under all conditions after the BOD containers had been left. On the other hand, in a case where the present activity improving agent was used, it was found that after the BOD containers had been left under many conditions, the BOD activity improved as compared with the BOD activity prior to leaving the BOD containers.

The following will specifically describe a case where L-ascorbic acid was used. In this case, one day after the BOD containers were left in an environment at a temperature of not lower than 40°C, three days after the BOD containers were left in an environment at a temperature of 30°C, and seven days after the BOD containers were left in an environment at a temperature of 20°C, the BOD activity increased by not less than approximately 2 times as compared with that prior to leaving the BOD containers.

The following will specifically describe a case where uric acid was used. In this case, one day after the BOD containers were left in the environment at a temperature of not lower than 40°C, the BOD activity increased by approximately 1.8 times or by approximately 2 times as compared with that prior to leaving the BOD containers. Further, three days after the BOD containers were left in the environment at a temperature of 30°C, and eight days after the BOD containers were left in the environment at a temperature of 20°C, the BOD activity increased by approximately 1.8 times as compared with that prior to leaving the BOD containers.

In both of the case where L-ascorbic acid was used and the case where uric acid was used, the BOD activity in the BOD containers left in an environment at a temperature of 5°C was not found to be increased to an extend that was achieved in a case where the BOD containers were left in an environment at a temperature of not lower than 10°C.

From the above results, it has been suggested that in the present activity improving method, it is preferable to leave the BOD solution in the environment at a temperature of not lower than 10°C. It has been also suggested that in a case where the BOD solution is left in a higher temperature environment, the BOD activity can be increased in a shorter period of time.

Similarly to Test Example 1, Test Example 2 can be also said to be implementation of the present activity improving method. Further, Test Example 2 can be also said to be production of the present BOD product. In addition, Test Example 2 can be also said to be implementation of a method for producing a highly active bilirubin oxidase in accordance with an embodiment of the present invention.

### (Test Example 3) Evaluation of ABTS initial absorbance (A₄₃₆) reduction rate of activity improving agent

The ABTS initial absorbance (A₄₃₆) reduction rate (%) was evaluated for the present activity improving agents and the comparative substances which were used in Test Example 1.

The following describes a specific evaluation method. ABTS employed was an ABTS manufactured by Roche Diagnostics K.K. Further, the ABTS was dissolved in a 50 mM glycine-KOH (pH 8.5) buffer so that the concentration of the ABTS became 2.0 mg/mL. An ABTS solution was thus prepared. The absorbance of the ABTS solution at a wavelength of 436 nm (ABTS initial absorbance (A₄₃₆)) was measured at an optical path length of 1.0 cm, by using a molecular absorption spectrometer Spectra Max Plus 384 (manufactured by Molecular Devices Japan K.K.). As a result the absorbance was approximately 1.3. Next, 5 µL of an activity improving agent solution or a comparative substance solution was added to 195 µL of the ABTS solution, so that a reaction solution for evaluating ABTS (hereinafter, also referred to as "reaction solution A") was prepared. Here, the amount of each of the above activity improving agents and comparative substances in the reaction solution A was set as follows: (a) the amount of the activity improving agent or the comparative substance was set to 1 mM in the reaction solution A, when the molecular weight of the activity improving agent or the comparative substance was evident as in a case where the activity improving agent or the comparative substance was made of a single compound; and (b) when the molecular weight of the activity improving agent or the comparative substance was made of a composition having an inevident molecular weight, the amount of the activity improving agent or the comparative substance was set such that the content of nitrogen element derived from the activity improving agent or the comparative substance was 0.011g in 100 mL of the reaction solution A. The final concentration of the activity improving agent or the comparative substance in the reaction solution A was shown in Table 7 in (a) "mM" for the activity improving agent or the comparative substance each having an evident molecular weight and (b) "percentage (%)" for the activity improving agent or the comparative substance each having an inevident molecular weight (amount (g) of the activity improving agent or the comparative substance relative to 100 mL of the reaction solution A).

Subsequently, immediately after the reaction solution A was prepared, that is, immediately after the activity improving agent solution or the comparative substance solution was added to the ABTS solution, the reaction solution A was reacted for 1 minute at 25°C. Immediately then, the absorbance of the reaction solution A at a wavelength of 436 nm was measured by using a molecular absorption spectrometer which is of the same type as that used for measurement of the absorbance of the ABTS solution. Further, 5 µL of distilled water, in place of the activity improving agent solution or the comparative substance solution, was added to 195 µL of the ABTS solution, so that another reaction solution A containing no activity improving agent or no comparative substance was prepared and used as a control (denoted as Without additive in Table 2 and Fig. 2). For the control, after the reaction solution A was reacted for 1 minute at 25°C, the absorbance of the reaction solution A at a wavelength of 436 nm was also measured by using the molecular absorption spectrometer. The ABTS initial absorbance (A₄₃₆) obtained as described above was compared with the absorbance at a wavelength of 436 nm obtained as described above for the reaction solution A. Then, calculation was carried out for an absorbance reduction rate (%)of the reaction solution A in one minute after addition of the activity improving agent or the comparative substance, with respect to the ABTS initial absorbance (A₄₃₆) (100%). The absorbance reduction rate (%) thus calculated was used as the ABTS initial absorbance (A₄₃₆) reduction rate (%). Table 7 and Fig. 2 show results of this calculation.

**[Table 7]**

| ABTS initial absorbance (A₄₃₆) reduction rate (%) of activity improving agent or comparative substance | | |
|---|---|---|
| Activity improving agent or comparative substance | Final concentration (%) | A₄₃₆ reduction rate (%) |
| DTT | 1 mM | 87.81 |
| Ammonium thioglycolate | 1 mM | 87.81 |
| Cysteamine hydrochloride | 1 mM | 87.81 |
| GMT | 1 mM | 87.81 |
| Sodium sulfite | 1 mM | 87.81 |
| Glutathione (reduced form) | 1 mM | 87.81 |
| L-cysteine hydrochloride | 1 mM | 87.81 |
| N - acetyl- L- cysteine | 1 mM | 87.81 |
| L-ascorbic acid | 1 mM | 87.81 |
| Trolox | 1 mM | 83.90 |
| Uric acid | 1 mM | 71.49 |
| Malt extract powder | 0.220(%) | 67.68 |
| Fish extract powder | 0.184(%) | 38.88 |
| Yeast extract powder | 0.100(%) | 27.17 |
| CSL | 0.158(%) | 26.52 |
| Meast P1G | 0.050(%) | 11.11 |
| Peptone A | 0.110(%) | 4.36 |
| Soybean Flour 88 | 0.100(%) | 3.79 |
| Yeast Extract | 0.050(%) | 3.74 |
| Particase | 0.110(%) | 0.59 |
| Glyoxylic acid | 1 mM | 0.33 |
| Hipolypepton | 0.100(%) | 0.29 |
| Peptone | 0.092(%) | 0.29 |
| Without additive | - | 0.00 |
| Urea | 1 mM | 0.00 |
| Ammonium sulfate | 1 mM | 0.00 |

Table 7 shows a reduction rate of the absorbance of the reaction solution A at a wavelength of 436 nm (ABTS initial absorbance (A₄₃₆) reduction rate (%)) in one minute after adding, to the solution A, the activity improving agent in accordance with an embodiment of the present invention or the comparative substance, relative to the absorbance of the ABTS solution at a wavelength of 436 nm (ABTS initial absorbance (A₄₃₆)). Fig. 2 shows the ABTS initial absorbance (A₄₃₆) reduction rate (%) of the activity improving agent in accordance with an embodiment of the present invention or the comparative substance, and specifically, shows, in a graph, the results shown in Table 7.

From Fig. 2, it has been found that the ABTS initial absorbance does not change in the reaction solution A without any additive and in the reaction solution A containing urea or ammonium sulfate. On the other hand, it has been found that the reaction solution A containing the present activity improving agent has an ABTS initial absorbance reduction rate of not less than 0.10%.

### (Test Example 4) Evaluation of DMBQ initial absorbance (A₂₅₆) reduction rate (%) of activity improving agent

The DMBQ initial absorbance (A₂₅₆) reduction rate (%) was evaluated for the present activity improving agents and the comparative substances which were used in Test Example 1. The activity improving agents and the comparative substances which were used in Test Example 4 were each a substance shown in Table 8 provided later.

The following describes a specific evaluation method. DMBQ employed was a DMBQ manufactured by Tokyo Chemical Industry Co., Ltd. First, 1 mM of a DMBQ solution was prepared. Measurement of an absorbance (A₂₅₆) of 1 mM of the DMBQ solution at a wavelength of 256 nm was measured at an optical path length of 1.0 cm, by using an ultra violet-visible spectrophotometer UV-160A (manufactured by Shimadzu Corporation). As a result, the absorbance was approximately 1.8. Next, to 890 µL of distilled water, (a) 100 µL of a 1 mM DMBQ solution and (b) 10 µL of the activity improving agent solution or the comparative substance solution were added, so that a reaction solution for evaluating DMBQ (hereinafter, also referred to as "reaction solution D") was prepared. Here, the amount of each of the above activity improving agents and comparative substances in the reaction solution D was set as follows: (a) the amount of the activity improving agent or the comparative substance was set to 0.1 mM in the reaction solution D, when the molecular weight of the activity improving agent or the comparative substance was evident as in a case where the activity improving agent or the comparative substance was made of a single compound; and (b) when the molecular weight of the activity improving agent or the comparative substance was made of a composition having an inevident molecular weight, the amount of the activity improving agent or the comparative substance was set such that the content of nitrogen element derived from the activity improving agent or the comparative substance was 0.0011g in 100 mL of the reaction solution D. The final concentration of the activity improving agent or the comparative substance in the reaction solution D was shown in Table 8 in (a) "mM" for the activity improving agent or the comparative substance each having an evident molecular weight and (b) "percentage (%)" for the activity improving agent or the comparative substance each having an inevident molecular weight (amount (g) of the activity improving agent or the comparative substance relative to 100 mL of the reaction solution D). Further, by using 10 µL of distilled water in place of the activity improving agent solution or the comparative substance solution, another reaction solution D was prepared. This reaction solution D was used as a control (denoted as Without additive in Table 3).

**[Table 8]**

| Final concentration of activity improving agent or comparative substance used in evaluation of DMBQ initial absorbance (A₂₅₆) reduction rate (%) | |
|---|---|
| Activity improving agent or comparative substance | Final concentration |
| DTT | 0.1 mM |
| Ammonium thioglycolate | 0.1 mM |
| Cysteamine hydrochloride | 0.1 mM |
| GMT | 0.1 mM |
| Sodium sulfite | 0.1 mM |
| Glutathione (reduced form) | 0.1 mM |
| L-cysteine hydrochloride | 0.1 mM |
| N-acetyl-L-cysteine | 0.1 mM |
| L-ascorbic acid | 0.1 mM |
| Trolox | 0.1 mM |
| Uric acid | 0.1 mM |
| Malt extract powder | 0.022% |
| Fish extract powder | 0.018% |
| Yeast extract powder | 0.010% |
| CSL | 0.016% |
| Meast P1G | 0.005% |
| Peptone A | 0.011% |
| Soybean Flour 88 | 0.010% |
| Yeast Extract | 0.005% |
| Particase | 0.011% |
| Glyoxylic acid | 0.1 mM |
| Hipolypepton | 0.010% |
| Peptone | 0.009% |

The absorbance of the reaction solution D at a wavelength of 256 nm was measured by using the ultra violet-visible spectrophotometer UV-160A at the following time points: (a) immediately after preparation of the reaction solution D (that is, immediately after the start of this test; denoted as 0 min in (a) of Fig. 3) and (b) every 10 seconds after the reaction solution D was left at 25°C. The absorbance of the reaction solution D at a wavelength of 256 nm immediately after preparation of the reaction solution D here was defined as the DMBQ initial absorbance (A₂₅₆). On the basis of measurement results, calculation was carried out for an absorbance reduction rate (%) of the reaction solution D in one minute after addition of the activity improving agent or the comparative substance of (after the start of the test) with respect to the DMBQ initial absorbance (A₂₅₆) (100%). The absorbance reduction rate (%) thus calculated was used as the DMBQ initial absorbance (A₂₅₆) reduction rate (%). Fig. 3 shows results of this calculation.

Fig. 3 shows the DMBQ initial absorbance (A₂₅₆) reduction rate (%) of the activity improving agent in accordance with an embodiment of the present invention or the comparative substance. Specifically, (a) of Fig. 3 shows a relative reduction rate (%) of absorbance of the reaction solution D at a wavelength of 256 nm. The relative reduction rate (%) of absorbance is a rate of absorbance after addition of the activity improving agent in accordance with an embodiment of the present invention or the comparative substance (after the start of this test), on the assumption that the absorbance immediately after the start of the test (0 minute) was 100%. (b) of Fig. 3 shows an absorbance reduction rate (%) (DMBQ initial absorbance (A₂₅₆) reduction rate (%))of the reaction solution D at a wavelength of 256 nm in one minute. The absorbance reduction rate (%) is a rate of absorbance after addition of the activity improving agent in accordance with an embodiment of the present invention or the comparative substance (after the start of this test), relative to the absorbance immediately after the start of the test (0 minute).

Note that Fig. 3 does not show the malt extract powder, the fish extract powder, the yeast extract powder, CSL, Meast P1G, Peptone A, Soybean Flour 88, Yeast Extract, Particase, Hipolypepton, and the peptone which were used in Test Example 4. This is because (1) these substances had no change from 100% in the relative reduction rate (%) ((a) of Fig. 3) of the absorbance of the reaction solution D at a wavelength of 256 nm and (2) the DMBQ initial absorbance (A₂₅₆) reduction rate (%) of each of these substances was 0.00% (that is, the detection limit or lower).

### Industrial Applicability

An embodiment of the present invention makes it possible to improve BOD activity. Therefore, the present invention can be suitably applied in a wide range of fields using a BOD, for example, in the field of hair dyeing, the field of clinical laboratories, the field of cosmetics, the field of enzyme fuel cells (e.g., enzymatic biofuel cells, and enzymatic batteries), and the field of food processing.

## Claims

1. A method for improving bilirubin oxidase activity, the method comprising the step of leaving, in a deoxygenated atmosphere, a bilirubin oxidase solution containing a bilirubin oxidase and an activity improving agent,
the activity improving agent is at least one substance selected from the group consisting of uric acid, glyoxylic acid, malt extract, fish extract, yeast extract, corn steep liquor, peptones, soybean flour, sodium sulfite, glutathione (reduced form), L-cysteine hydrochloride, N-acetyl-L-cysteine, L-ascorbic acid, and Trolox, said activity improving agent having an 2,2'-azinobis(3-ethylbenzothiazoline-6-sulfonic acid (ABTS) initial absorbance (A₄₃₆) reduction rate (%) of not less than 0.10% and a 2,5-dimethyl-1,4-benzoquinone (DMBQ) initial absorbance (A₂₅₆) reduction rate (%) of not more than 10.00%.

2. The method as set forth in claim 1, wherein the bilirubin oxidase solution is left in an environment at a temperature of not lower than 10°C.

3. A bilirubin oxidase product comprising:
a container; and
a bilirubin oxidase and an activity improving agent in the container,
the container having an inner atmosphere which is deoxygenated, and
the activity improving agent is at least one substance selected from the group consisting of uric acid, glyoxylic acid, malt extract, fish extract, yeast extract, corn steep liquor, peptones, soybean flour, sodium sulfite, glutathione (reduced form), L-cysteine hydrochloride, N-acetyl-L-cysteine, L-ascorbic acid, and Trolox, said activity improving agent having an 2,2'-azinobis(3-ethylbenzothiazoline-6-sulfonic acid (ABTS) initial absorbance (A₄₃₆) reduction rate (%) of not less than 0.10% and a 2,5-dimethyl-1,4-benzoquinone (DMBQ) initial absorbance (A₂₅₆) reduction rate (%) of not more than 10.00%.

4. The bilirubin oxidase product as set forth in claim 3, wherein the bilirubin oxidase and the activity improving agent are in a solution state.

## Patentansprüche

1. Verfahren zur Verbesserung der Bilirubinoxidase-Aktivität, wobei das Verfahren den Schritt umfasst, eine Bilirubinoxidase-Lösung, die eine Bilirubinoxidase und ein die Aktivität verbesserndes Mittel enthält, in einer sauerstoffarmen Atmosphäre zu belassen,
wobei das die Aktivität verbessernde Mittel mindestens eine Substanz ist, die aus der Gruppe ausgewählt ist, die aus Harnsäure, Glyoxylsäure, Malzextrakt, Fischextrakt, Hefeextrakt, Maisquellwasser, Peptonen, Sojamehl, Natriumsulfit, Glutathion (reduzierte Form), L-Cystein-Hydrochlorid, N-Acetyl-L-Cystein, L-Ascorbinsäure und Trolox besteht, wobei das die Aktivität verbessernde Mittel eine 2,2'-Azinobis(3-ethylbenzothiazolin-6-sulfonsäure(ABTS)-Anfangsabsorbanz(A₄₃₆)-Verringerungsrate (%) von nicht weniger als 0,10 % und eine 2,5-Dimethyl-1,4-benzochinon(DMBQ)-Anfangsabsorbanz(A₂₅₆)-Verringerungsrate (%) von nicht mehr als 10,00 % aufweist.

2. Verfahren gemäß Anspruch 1, wobei die Bilirubinoxidase-Lösung in einer Umgebung bei einer Temperatur von nicht weniger als 10°C belassen wird.

3. Bilirubinoxidase (umfassendes) Produkt, umfassend:
einen Behälter und
eine Bilirubinoxidase und ein die Aktivität steigerndes Mittel in dem Behälter,
wobei der Behälter eine innere Atmosphäre aufweist, die sauerstoffarm ist, und
wobei das die Aktivität verbessernde Mittel mindestens eine Substanz ist, die aus der Gruppe ausgewählt ist, die aus Harnsäure, Glyoxylsäure, Malzextrakt, Fischextrakt, Hefeextrakt, Maisquellwasser, Peptonen, Sojamehl, Natriumsulfit, Glutathion (reduzierte Form), L-Cystein-Hydrochlorid, N-Acetyl-L-Cystein, L-Ascorbinsäure und Trolox besteht, wobei das die Aktivität verbessernde Mittel eine 2,2'-Azinobis(3-ethylbenzothiazolin-6-sulfonsäure(ABTS)-Anfangsabsorbanz(A₄₃₆)-Verringerungsrate (%) von nicht weniger als 0,10 % und eine 2,5-Dimethyl-1,4-benzochinon(DMBQ)-Anfangsabsorbanz(A₂₅₆)-Verringerungsrate (%) von nicht mehr als 10,00 % aufweist.

4. Bilirubinoxidase (umfassendes) Produkt gemäß Anspruch 3, wobei die Bilirubinoxidase und das die Aktivität verbessernde Mittel sich in einem gelösten Zustand befinden.

## Revendications

1. Procédé d'amélioration de l'activité de la bilirubine oxydase, le procédé comprenant l'étape consistant à laisser, dans une atmosphère désoxygénée, une solution de bilirubine oxydase contenant une bilirubine oxydase et un agent améliorant l'activité,
l'agent améliorant l'activité est au moins une substance choisie dans le groupe constitué par l'acide urique, l'acide glyoxylique, un extrait de malt, un extrait de poisson, un extrait de levure, une liqueur de macération de maïs, des peptones, une farine de soja, le sulfite de sodium, le glutathion (forme réduite), le chlorhydrate de L-cystéine, la N-acétyl-L-cystéine, l'acide L-ascorbique et le Trolox, ledit agent améliorant l'activité ayant un taux de réduction (%) d'absorbance initiale d'acide 2,2'-azinobis(3-éthylbenzothiazoline-6-sulfonique (ABTS) (A₄₃₆) de pas moins de 0,10 % et un taux de réduction (%) d'absorbance initiale de 2,5-diméthyl-1,4-benzoquinone (DMBQ) (A₂₅₆) de pas plus de 10,00 %.

2. Procédé tel que décrit dans la revendication 1, dans lequel la solution de bilirubine oxydase est laissée dans un environnement à une température de pas moins de 10 °C.

3. Produit de bilirubine oxydase comprenant :
un récipient ; et
une bilirubine oxydase et un agent améliorant l'activité dans le récipient,
le récipient ayant une atmosphère interne qui est désoxygénée, et
l'agent améliorant l'activité est au moins une substance choisie dans le groupe constitué par l'acide urique, l'acide glyoxylique, un extrait de malt, un extrait de poisson, un extrait de levure, une liqueur de macération de maïs, des peptones, une farine de soja, le sulfite de sodium, le glutathion (forme réduite), le chlorhydrate de L-cystéine, la N-acétyl-L-cystéine, l'acide L-ascorbique et le Trolox, ledit agent améliorant l'activité ayant un taux de réduction (%) d'absorbance initiale d'acide 2,2'-azinobis(3-éthylbenzothiazoline-6-sulfonique (ABTS) (A₄₃₆) de pas moins de 0,10 % et un taux de réduction (%) d'absorbance initiale de 2,5-diméthyl-1,4-benzoquinone (DMBQ) (A₂₅₆) de pas plus de 10,00 %.

4. Produit de bilirubine oxydase tel que décrit dans la revendication 3, dans lequel la bilirubine oxydase et l'agent améliorant l'activité sont dans un état de solution.
